# EUROPEAN PATENT SPECIFICATION

(11) **EP 0 957 110 B1**
(45) Date of publication and mention of the grant of the patent: **06.12.2006**
(21) Application number: 99109174.5
(22) Date of filing: 10.05.1999
(51) Int. Cl.: C07K 14/47, C07K 1/32, G01N 33/68, A61K 38/17

(54) **Lyophilized tubulins**
Lyophilisierte Tubuline
Tubulines lyophilisées

(30) Priority: 14.05.1998 US 85382 P
(43) Date of publication of application: 17.11.1999
(73) Proprietor: Cytoskeleton, Inc., Denver, CO 80223 (US)
(72) Inventor: Middleton, Kim Ph.D., c/o Cytoskeleton, Inc., Denver, CO 80206 (US); Ashley, Davis Ph.D., c/o Cytoskeleton, Inc., Denver, CO 80206 (US)
(74) Representative: Lee, Nicholas John

(56) References cited:
- FR-A- 2 349 335
- SOIFER ET AL.: "Enzymatic activity in tubulin preparations ...." BIOCHEMICA ET BIOPHYSICA ACTA, vol. 271, 1972, pages 182-192, XP008004530
- WEISENBERG ET AL.: "Aggregation of microtuble subunit protein. Effects of divalent cations, colchicine and vinblastine" BIOCHEMISTRY, vol. 9, no. 21, 1970, pages 4110-4116, XP001073954
- RINGEL ET AL.: "Effect of alkaline pH on taxol-microtubule interactions" THE JOURNAL OF PHARMACOLOGY AND EXPERIMENTAL THERAPEUTICS, vol. 259, no. 2, 1991, pages 855-860, XP008004529
- SHELANSKI ET AL.: "Microtubule assembly in the absence of added nucleotides" PROCEEDINGS OF THE NATIONAL ACADEMY OF SCIENCES, US, vol. 70, no. 3, March 1973 (1973-03), pages 765-768, XP008002267

## Description

### Field of the Invention

The present invention relates to a method for the isolation of lyophilized active tubulin, to a lyophilized active tubulin protein obtainable by this method which polymerises upon reconstitution, and to the use of lyophilized active tubulin. More particularly, the present invention relates to the diagnostic applications of lyophilized active tubulin, and the use of lyophilized active tubulins in the fields of drug discovery and research.

### Discussion of the Background Art

Tubulin is an essential intracellular protein that is necessary for mitosis, transport of intracellular material, cell structure, and cell motility. Tubulin is composed of a heterodimer of two closely related 55 Kilodalton proteins called alpha and beta tubulin. These two proteins are encoded by separate genes or small gene families, whose sequences are highly conserved throughout the eukaryotic kingdom.

Tubulin polymerizes to form structures called microtubules. When tubulin polymerizes it initially forms protofilaments. Microtubules consist of 13 protofilaments and are 25 nm in diameter, each µm of microtubule length being composed of 1650 tubulin heterodimers. Microtubules are highly ordered fibres that have an intrinsic polarity. There is a dynamic flux between microtubules and tubulin. When this equilibrium is perturbed by anti-tubulin agents like paclitaxel (taxol), cells will arrest in mitosis and eventually die. (Schiff *et al*.,1980, *Proceedings of the National Academy of Sciences USA*, **77**, 1561-1565.) This is the mechanistic basis of how anti-tumor (taxol, vinblastine, and nocodozole), anti-fungal (benomyl) and anthelmintic (mebendazole) agents interact with tubulin. Vinblastine, paclitaxel (taxol TM Bristol Myers-Squibb Co.), and nocodozole bind to tubulin and thus inhibit mitosis (Weisenberg, R.C., and S.N. Timasheff, 1970, *Biochemistry*, **21**, 4110-4116; Schiff *et al*.,1980.) These agents were identified through cell or organism based screens and were only later found to interact with tubulin.

Different forms of tubulin have been isolated. These include a microtubule associated protein (MAP)-rich tubulin that is 50% to 97% purified (Shelanski, M.L., Gaskin, F., and C.R. Cantor, 1973, *Proceedings of the National Academy of Sciences USA*, **70**, 765-768), highly purified (97% to 99.99% or apparently 100% purified by silver stain or coomassie-blue stained SDS-PAGE) tubulin, *e.g*., Phospho-cellulose purified tubulin (Lee, J.C., Tweedy, N., S.N. Timasheff, 1978, *Biochemistry*, **17**(14), 2783-2790), tubulin from crude cancer cell line extracts (Weatherbee, J.A., Luftig, R.B., R.R. Weihing, 1980, *Biochemistry,* **19**(17), 4116-4123), tubulin isolated from higher eukaryotes and their cell lines (Weatherbee *et al*. 1980), tubulin isolated from fungi and yeasts and their cell lines (Davis, A., Sage, C.R., Dougherty, C., K.W. Farrell, 1993, *Biochemistry*, **32**, 8823-8835), tubulin isolated from parasitic organisms or their cell lines (Dawson, PJ., Gutteridge, W.E., K. Gull, 1983, *Molecular and Biochemical Parasitology,* **7**(3), 267-277), and tubulins isolated from recombinant systems and recombinant organisms (Davis, A., Sage, C.R., Dougherty, C., K.W. Farrell, 1994, *Science*, **264**, 839-842.)

Although tubulin is the target of several anti-tumor, anti-fungal and anthelmintic (anti-parasitic) agents, it has not been utilized as a target in high through-put drug screening programs, diagnostic screens, or therapy because of its lability. Tubulin is unstable and will denature, becoming inactive within two days at 4°C (Shelanski *et al.,* 1973.) Attempts have been made to create lyophilized forms of tubulin that are stable at room temperature. Since the first purification of tubulin by Weisenberg and Timasheff in 1970, several attempts at tubulin lyophilization have been recorded. The first by Weisenberg and Timasheff in 1970 involved crude lyophilizations on impure tubulin preparations at 97% purity (Weisenberg *et al.,* 1970). This study did not result in assembly competent tubulin. The second attempt in 1972 by Soifer *et al*. involved the reconstitution of tubulin into physiological buffer but resulted in no microtubule formation (Soifer, D., Laszlo A.H., J.M. Scotto, 1972, *Biochimica et Biophysica Acta*, **271**, 182-192). Thus, the resulting protein was, incompetent for assembly and, therefore, inactive. Following Weisenberg *et al.* and Soifer *et al*. the dogma in tubulin biochemistry has been that highly purified tubulin cannot be lyophilized with high activity. In 1996, Sigma Chemical Company, St. Louis, MO (Sigma) introduced a 30-40% crude preparation of tubulin (Tubulin, *Sigma Chemical Company 1996 Catalog*, Catalog No. T4925). The product profile for T4925 (Lot. No. 87H4024) states that microtubules are formed upon reconstitution. However, comparative laboratory studies between T4925 and the lyophilized tubulin of the present invention disclosed herein have shown that the tubulin has very low activity as tested by a polymerization assay and very few microtubules are visible by electron microscopy. Thus, even if the Sigma formulation is sometimes successful at producing active tubulin, different batches vary widely and are inconsistent at best.

After the failures to successfully lyophilize tubulin in 1970 and 1972, Shelanski *et al.* in 1973 demonstrated the frozen liquid approach to tubulin storage. In this method, tubulin solution is rapidly frozen in liquid nitrogen and is stored at temperatures below -70°C. Presently, this is the accepted method for storage of tubulin. This method is unsuitable for high through-put screening and other uses because retrieving the vials requires dexterity that cannot be automated easily. Another practical problem is the reproducibility of this method since the activity of preparations varies widely during the length of storage.

Prior to the invention disclosed herein, there remains a need for:
(1) lyophilized active tubulin that is highly active, stable and capable of providing reproducible results;
(2) lyophilized active tubulin that is competent of microtubule assembly;
(3) a lyophilization method for pure tubulin whereby tubulin can be lyophilized with high activity;
(4) appropriate vessels for lyophilization of tubulin so that the reconstituted form may be used for different applications; and
(5) methods of high through-put screening methods for tubulin and tubulin ligands.

### Summary of the Invention

In a first aspect, the present invention provides a method for the isolation of lyophilized active tubulin comprising the steps of:
(a) running multiple cycles of polymerisation and depolymerisation of tubulin;
(b) conducting differential sedimentation centrifugation to create a pellet of active tubulin;
(c) resuspending the pellet in a lyophilization buffer at a tubulin concentration of between 1 µg/ml and 200 mg/ml, the lyophilization buffer comprising (a) a buffer, a sugar, a carbohydrate polymer, a salt, a nucleotide and a protein or (b) distilled water;
(d) freezing the resuspended pellet in liquid nitrogen;
(e) lyophilizing the frozen resuspended pellet at a temperature of 10 °C or below.

In a second aspect, the present invention provides a lyophilized active tubulin protein obtainable by the method of the first aspect, which polymerises upon reconstitution. The lyophilized active tubulin protein can be a modified tubulin, a recombinant tubulin, a cell line derived tubulin, or an isolated tubulin from tissues and organs.

In a third aspect, the present invention provides a microtubule polymerization assay for lyophilized active tubulin comprising the following steps: (a) warming lyophilized active tubulin protein obtainable by the method of the first aspect to 37 °C in a spectrophotometer in the presence of a reconstitution buffer; and (b) measuring the optical density at O.D. 340 nm over a time period.

In a fourth aspect, the present invention provides a diagnostic assay for tubulin ligands comprising the following steps: (a) adding the tubulin ligand in reconstitution buffer to a lyophilized tubulin protein obtainable by the method of the first aspect; and (b) measuring the optical density at O.D. 340 nm over a time period.

In a fifth aspect, the present invention provides a drug discovery process comprising the following steps: (a) reconstituting lyophilized active tubulin obtainable by the method of the first aspect in a reconstitution buffer; (b) incubating the reaction (at 24 °C or 37 °C) and measuring at O.D. 340nm over 60 minutes; and (c) comparing control and test samples for differences in polymerization.

In a sixth aspect, the present invention provides a drug discovery process comprising the following steps: (a) reconstituting lyophilized active tubulin obtainable by the method of the first aspect in a vessel that supports high through-put drug screening; (b) incubating the reaction and measuring at O.D. 340 nm; and (c) comparing control and test samples for differences in polymerization kinetics.

In a seventh aspect, the present invention provides a method for detecting the presence of an analyte in solution comprising the following steps: (a) adding the analyte to lyophilized tubulin protein obtainable by the method of the first aspect; and (b) measuring the optical density at O.D. 340 nm over a time period.

In an eighth aspect, the present invention provides a method of drug discovery which uses lyophilized active tubulin obtainable by the method of the first aspect as a component of a lyophilized mixture of proteins.

The present invention relates to a method of making a formulation of tubulin that is stable, i.e., it will not denature and will maintain its activity, for greater than one year at room temperature, or alternatively for greater than five years at 4°C. This tubulin formulation is active once reconstituted and is capable of polymerizing to form microtubules. Currently available lyophilized forms of tubulin are impure (≤ 40 % purity), labile and unable to form microtubules. The level of stability provided by the lyophilized form of tubulin made by the method of the current invention will greatly increase the use of tubulin for applications such as research, drug discovery, diagnostics and therapy. Research applications include the possible use of the lyophilized active tubulin of the present invention to solve the three dimensional structure of tubulin (Nogales, E., Wolf, S.G., K.H. Downing, 1998, *Nature,* **391**(6663), 199-203). The tubulin for the study conducted by Nogales *et al*., 1998, was supplied by Cytoskeleton, Inc. In the fifth, sixth and eighth aspects of the invention, drug discovery can be facilitated using the formulation of tubulin described in the invention as a substrate in high through-put screens for the development of new tubulin ligands. Diagnostic applications according to the fourth aspect of the invention include the rapid assay of anti-tumor agents, *e.g*., paclitaxel, and anthelmintic agents, *e.g*., mebendazole. Potential therapeutic applications include the use of lyophilized tubulin in conjunction with or without anti-tubulin ligands in a complex encapsulated within a liposome. The liposome can serve as a vehicle of drug delivery.

The methods of lyophilization and reconstitution described herein are breakthroughs in tubulin and tubulin ligand research and allow greater reproducibility and stability of this labile protein. Upon lyophilization, this method results in a highly active form of lyophilized tubulin that can be used with different isotypes, types, modified forms, purities, and recombinant forms of tubulin. Thus, this process is widely applicable to all forms of tubulin including microtubules formed from tubulin, MAP-rich tubulin (50% to 97% purified) (Shelanski *et al.* 1973), 97% to greater than 99% purified tubulin (e.g., Phospho-cellulose purified tubulin, Lee *et al*., 1978), tubulin in crude extracts (e.g., cancer cell line extracts), tubulin isolated from higher eukaryotes and their cell lines (Weatherbee *et al*. 1980; Morejohn and Fosket, 1982), tubulin isolated from fungi and yeasts and their cell lines (Davis *et al.* 1993), tubulin isolated from parasitic organisms or their cell lines (Dawson *et al.* 1983), and tubulins isolated from recombinant systems and recombinant organisms (Davis *et al.* 1994, Lubega *et al*. 1993).

The method of the present invention may be conducted in different vessels since different applications of lyophilized tubulin require appropriate vessels for lyophilization and reconstitution. This includes single vials for all applications, wells in multi-well plates for high through-put screening and diagnostics, glass slides for microscopy research and diagnostics, solid supports like dip sticks, filters, etc., frozen drops of liquids that may be lyophilized for large quantity therapeutic applications, and any new vessels such as micro and nano-sized reaction chambers that may be available in the future.

In the microtubule polymerization assay of the third aspect of the invention, the optical density at O.D. _{340 nm} is measured kinetically over time to determine polymerization and depolymerization. This kinetic assay can also be conducted at different temperatures to determine the stability of various forms of tubulin in comparison to the stable, active form of lyophilized tubulin of the present invention.

To facilitate the use of lyophilized active tubulin for the diagnostic application of assaying effective concentrations of anti-tumor agents like paclitaxel or anthelmintic agents like mebendazole, the fourth aspect of the present invention provides a high-throughput assay designed to measure anti-tubulin ligands. This assay can be used to measure ligand concentrations in body fluids like serum, urine, etc*.*, fermentor broths, and to understand steps in the synthesis of ligands.

Potential therapeutic applications involving the use of lyophilized active tubulin may include, for example, the encapsulation within a microsomal liposome of a complex of tubulin and tubulin ligands to aid in drug delivery mechanisms.

### Description of the Drawings

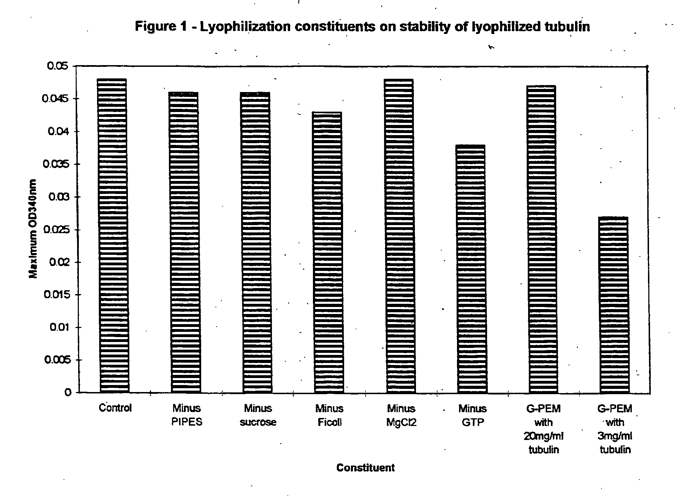

**Fig. 1** - illustrates the effect of lyophilization constituents on the short term stability of lyophilized tubulin. Procedures for lyophilization of MAP-rich tubulin are described below. The dried powder was resuspended at 1mg/ml protein in G-PEM and incubated at 24°C for 1hr. The optical density difference was determined between T=0 and T=60 minutes and that is plotted on the chart.

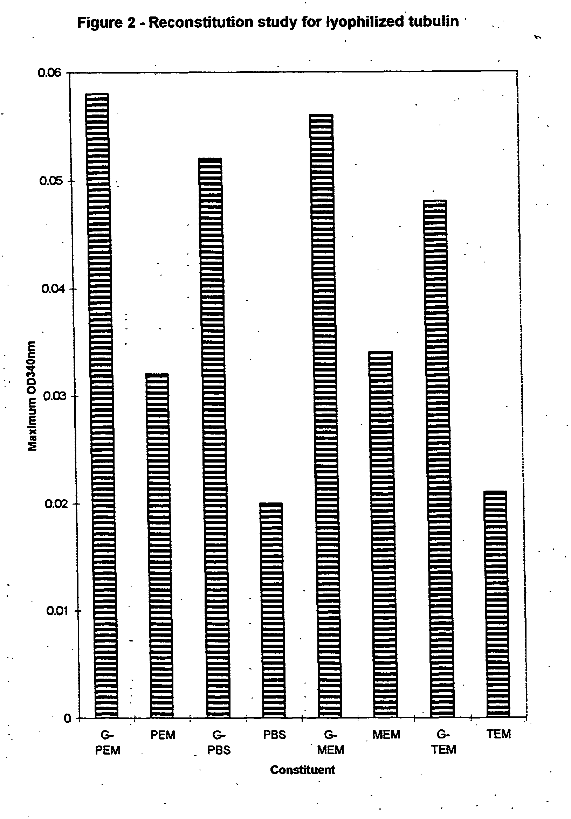

**Fig. 2** - Shows the reconstitution study for lyophilized tubulin. Procedures for lyophilization of MAP-rich tubulin are described below and the optimal buffer was used for all samples. The dried powder was resuspended at 1mg/ml protein in the noted buffers and incubated at 24°C for 1hr. The optical density difference was determined between T=0 and T=60 minutes and that is plotted on the chart.

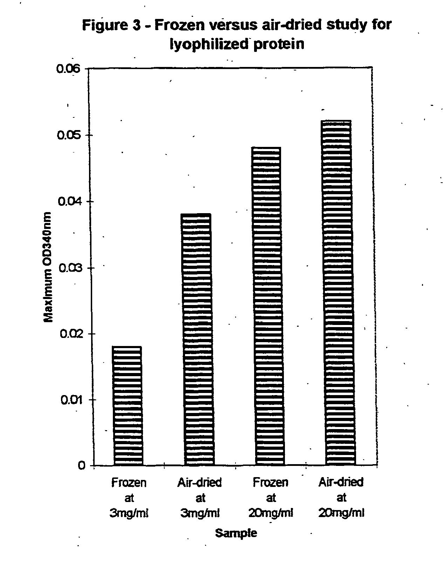

**Fig. 3** - Is a comparative illustration of a frozen vs. air-dried study for lyophilized tubulin. Procedures for lyophilization of MAP-rich tubulin are described below and the optimal buffer was used for all samples. The dried powder was resuspended at 1mg/ml protein in the G-PEM buffer and incubated at 24°C for 1hr. The optical density difference was determined between T=0 and T=60 minutes and the Maximum OD are plotted on the chart.

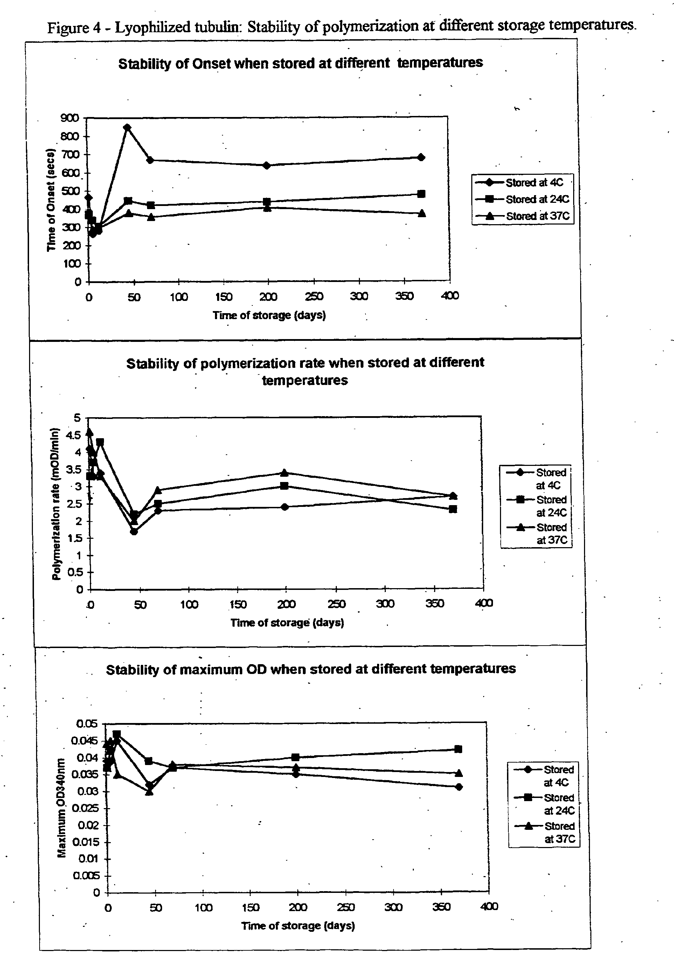

**Fig. 4** - illustrates the stability of lyophilized active tubulin over time compared using maximum polymerization measured at O.D.₃₄₀ₙₘ. Procedures for lyophilization of MAP-rich tubulin are described below and the optimal buffer was used for all samples. The dried powder was resuspended at 1mg/ml protein in the G-PEM buffer and incubated at 24°C for 1hr. The optical density difference was determined between T=0 and T=60 minutes and the Onset (time to pass a certain OD, which = 0.1x the maximum OD of the fully active protein), Polymerization rate (mOD/mm) and the Maximum OD are plotted on the charts.

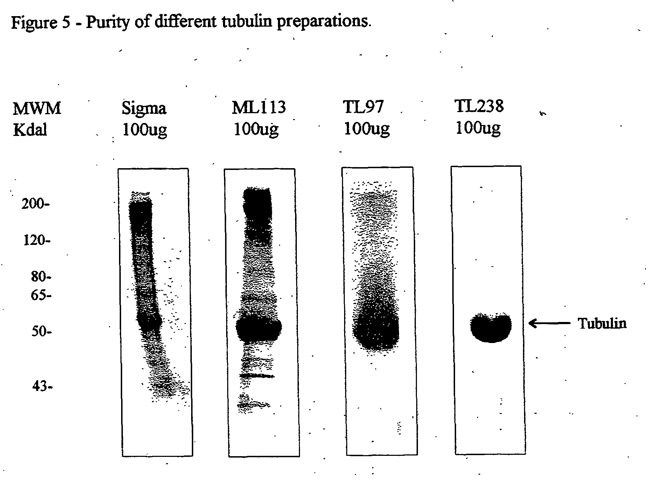

**Fig. 5** - Compares the estimated tubulin purity of Sigma T4925 tubulin with lyophilized active tubulin at 70%, 97% and >99% purity using SDS-PAGE (coomassie-blue stain). Samples of T4925 (Sigma Chemical Co.), ML113, TL97 and TL238 (Cytoskeleton Inc.) were run on a 10% polyacrylamide gel and stained with coomassie blue. 100ug of protein was loaded for each sample. Estimated purity with respect to tubulin is 30-40% for T4925,50-70% for ML113, 95-99% for TL97 and >99% for TL238.

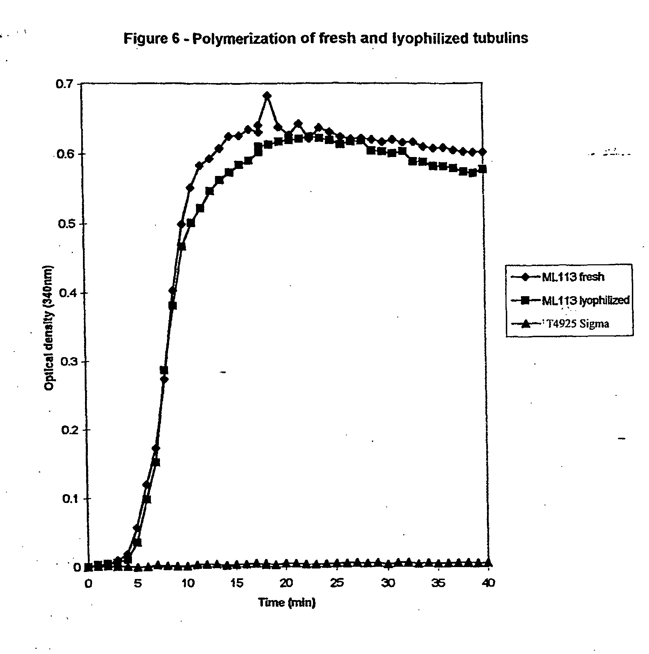

**Fig. 6** - Compares the polymerization of Sigma T4925 tubulin, freshly purified tubulin and lyophilized active tubulin. Procedures for lyophilization of MAP-rich tubulin are described below and the optimal buffer was used for all samples. The dried powder was resuspended at 5mg/ml protein in 4°C for 1h. The optical density 340nm was determined between T=O and T=60min.

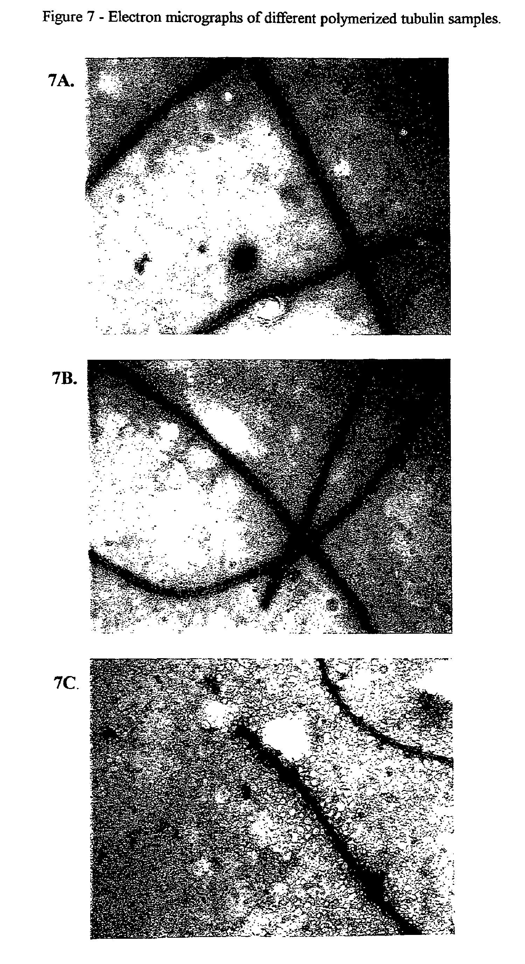

**Fig. 7** - A illustrates a negative stain electron photomicrograph (28,000 magnification) of microtubules from fresh tubulin. Electron microscopy was performed by uranyl acetate negative staining of tubulin samples incubated at 37°C for 1h. Magnification is x28000. Procedures for lyophilization of MAP-rich tubulin are described below and the optimal buffer was used for all samples. The dried powder was resuspended to 1mg/ml protein in the G-PEM buffer. Tubulin from Sigma Chemical Co. was prepared in accordance with the company's information sheet provided with the product.
**Fig. 7a** - Freshly prepared MAP-rich tubulin
**Fig. 7b** - Illustrates a negative stain electron photomicrograph (28,000 magnification) of microtubules from lyophilized active tubulin. Lyophilized MAP-rich tubulin.
**Fig. 7c** - Shows an electron micrograph of Sigma's tubulin preparation after incubation at 37°C for 45 min. (28,000 magnification). T4925 tubulin from Sigma Chemical Co.

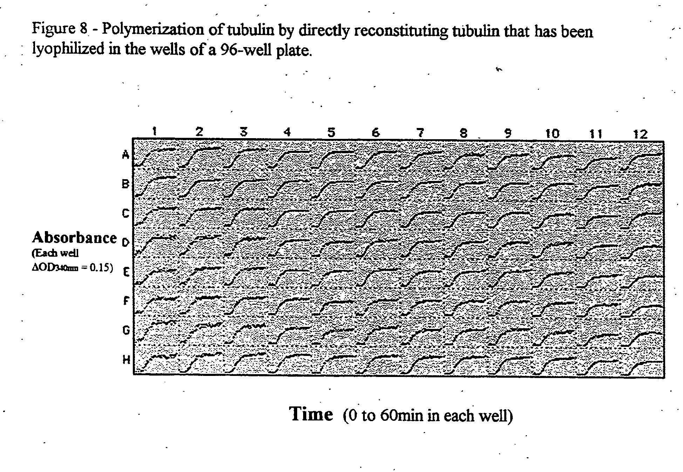

**Fig. 8** - Shows sample results of a 96 well plate assay with lyophilized tubulin. Procedures for lyophilization of MAP-rich tubulin are described below and the optimal buffer was used for all samples. The dried powder was resuspended to 1mg/ml protein in the G-PEM buffer and incubated at 24°C for 1h. The optical density was determined between T=O and T=60min.

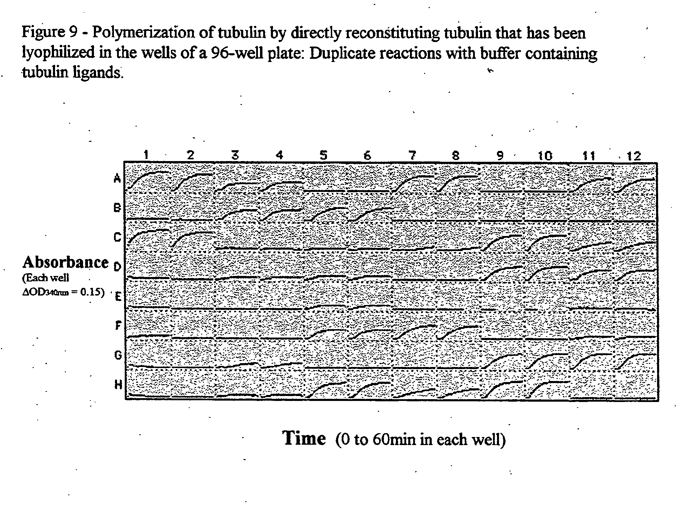

**Fig. 9** - Shows sample results of a 96 well plate assay with lyophilized tubulin and duplicate reactions. Procedures for lyophilization of MAP-rich tubulin are described below and the optimal buffer was used for all samples. The dried powder was resuspended to 1mg/ml protein in the G-PEM buffer that contained different tubulin ligands. The reactions were incubated at 24°C and the optical density was determined between T=O and T=60min. Wells A1, A2 - duplicate control reactions; wells B1, B2- Duplicate 3uM nocodazole reactions, and wells C1, C2 - Duplicate 3uM paclitaxel reactions. Other duplicate wells contained buffer with a random concentration of nocodazole between 0- 30uM.

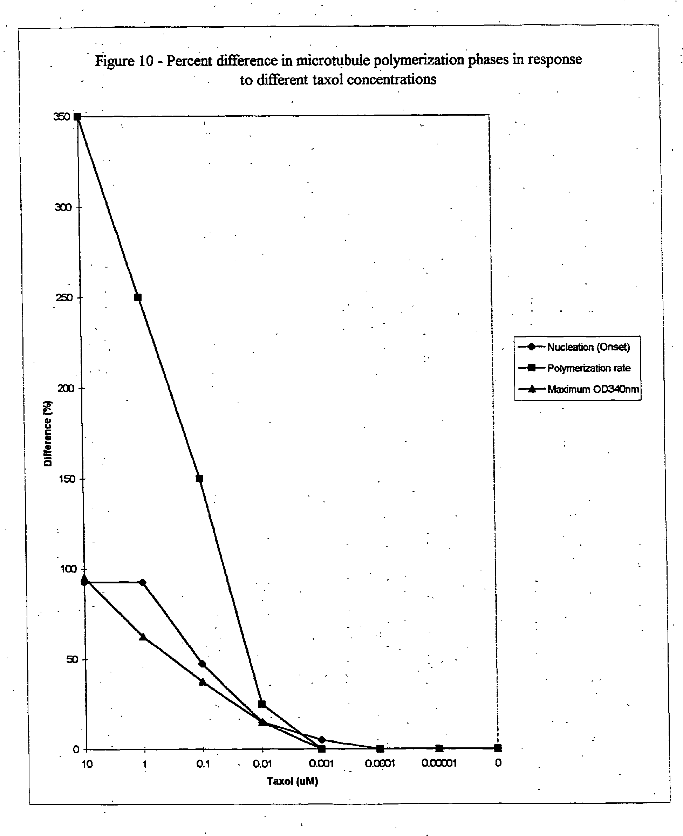

**Fig. 10** - Shows the Effect of taxol concentration on the polymerization rate of a prepared sample (No. ML113) of lyophilized active tubulin. Similar protocols were performed as in Fig. 9 legend except that paclitaxel was present at different concentrations and assays were performed in triplicate. Average nucleation (Onset) times, polymerization rates and maximum OD340nm ' s were compared to the control reactions (no drug) using CytoDYNAMIX Screen 1M data analysis package (Cytoskeleton, Inc.).

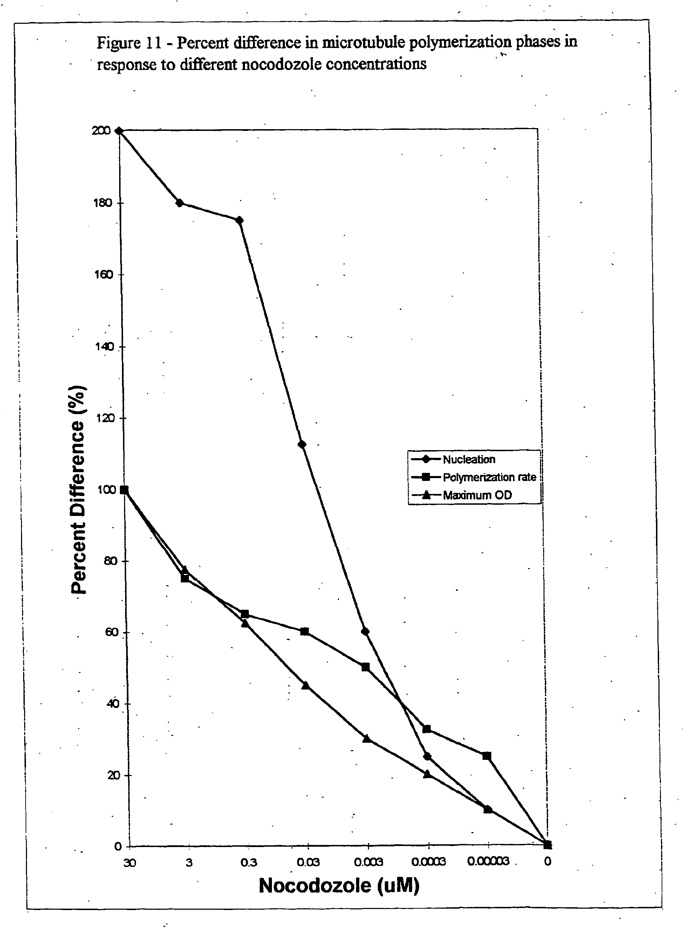

**Fig. 11** - Shows the Effect of nocodozole concentration on the polymerization rate of a prepared sample (No. ML113) of lyophilized active tubulin. Similar protocols were performed as in Fig. 9 legend except that nocodazole was present at different concentrations and assays were performed in triplicate. Average nucleation (Onset) times, polymerization rates and maximum OD340nm's were compared to the control reactions (no drug) using CytoDYNAMIX Screen ™ data analysis package (Cytoskeleton, Inc.).

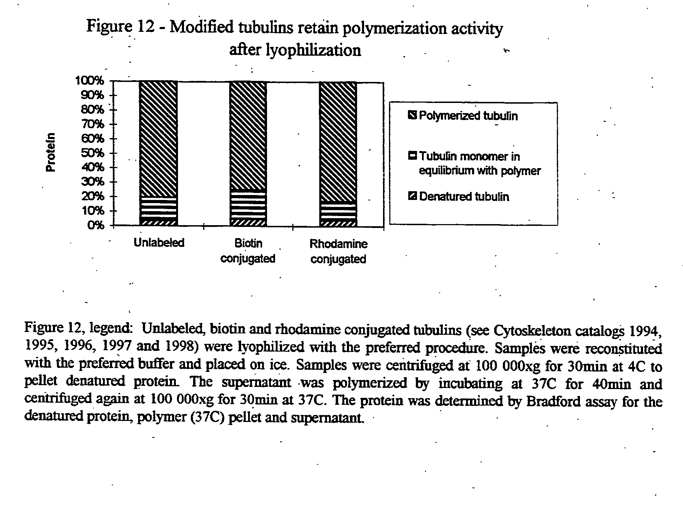

**Fig. 12** - Illustrates how modified tubulins retain polymerization activity after lyophilization. Unlabeled, biotin and rhodamine conjugated tubulins (see Cytoskeleton catalogs 1994, 1995, 1996, 1997 and 1998) were lyophilized with the preferred procedure. Samples were reconstituted with the preferred buffer and placed on ice. Samples were centrifuged at 100 000xg for 30min at 4°C to pellet denatured protein. The supernatant was polymerized by incubating at 37°C for 40min and centrifuged again at 100 000xg for 30 min at 37°C for 40 min and centrifuged again at 100 000xg for 30min at 37°C. The protein was determined by Bradford assay for the denatured protein, polymer (37°C) pellet and supernatant.

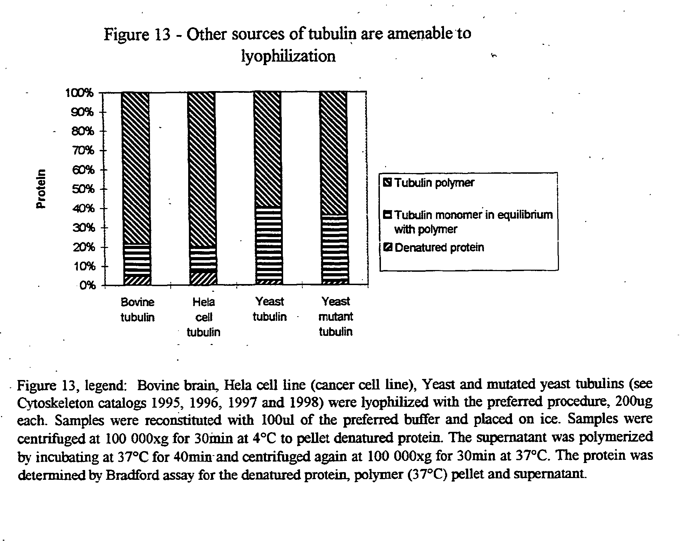

**Fig. 13** - Illustrates how other sources of tubulin are amenable to lyophilization. Bovine brain, Hela cell line (cancer cell line), Yeast and mutated yeast tubulins (see Cytoskeleton catalogs 1995, 1996, 1997 and 1998) were lyophilized with the preferred procedure, 200ug each. Samples were reconstituted with 100ul of the preferred buffer and placed on ice. Samples were centrifuged at 100 000xg for 30 mm at 4°C to pellet denatured protein. The supernatant was polymerized by incubating at 37°C for 40min and centrifuged again at 100 000xg for 30 min at 37°C. The protein was determined by Bradford assay for the denatured protein, polymer (37°C) pellet and supernatant.

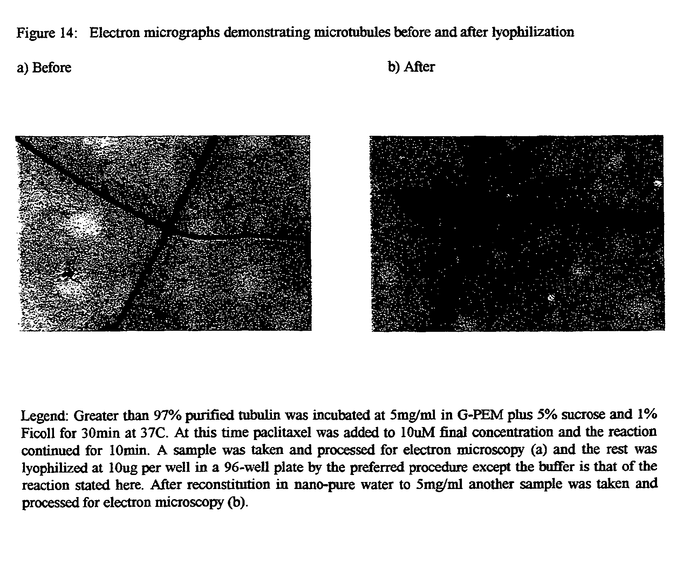

**Fig. 14** - Shows Microtubules before and after lyophilization. Greater than 97% purified tubulin was incubated at 5mg/ml in G-PEM plus 5% sucrose and 1% Ficoll for 30min at 37°C. At this time paclitaxel was added to 10uM final concentration and the reaction continued for 120min. A sample was taken and processed for electron microscopy (a) and the rest was lyophilized at 10ug per well in a 96-well plate by the preferred procedure except the buffer is that of the reaction stated here. After reconstitution in nano-pure water to 5mg/ml another sample was taken and processed for electron microscopy (b).

### Detailed Description of the Invention

The present invention relates to a method of making a formulation of lyophilized tubulin that is stable for greater than one year at room temperature, or alternatively for greater than five years at 4°C. This tubulin formulation is active once reconstituted and is capable of polymerizing to form microtubules. The reconstituted tubulin is then tested for biological activity using the microtubule polymerization assay of the third aspect of the present invention. This assay has been optimized to measure differences between the control and test reactions as detailed in the copyrighted manual for high through put screens called "CytoDYNAMIX Screen^{™}." Microtubule formation is further tested by electron microscopy. Comparative studies between the lyophilized tubulin product, Catalog No. T4925, sold by Sigma Chemical Company, and the lyophilized tubulin of the present invention show that T4925 microtubules are much less abundant with the T4925 product and the microtubules that do form are coated with denatured protein aggregates that suppress its biological activity and dynamic nature.

### (1) Isolation of Tubulin

Tubulin can be isolated from most eukaryotic and some recombinant prokaryotic sources by standard methods. Various degrees of purity are produced by these methods. (Weisenberg and Timasheff, 1970; Weatherbee *et al*. 1980; Davis *et al.* 1993; Barnes, G., Louie, J.M., D. Botstein, 1992, *Biochemistry*, **32,** 8823-8835; Lubega, G.W., Geary, T.G., Klein, R.D., R.K. Prichard, 1993, *Mol. Biochem. Parasitol*., **62**, 281-292.) Tubulin is isolated as MAP-rich bovine brain tubulin (Shelanski *et al*. 1973) by three cycles of polymerization and depolymerization, greater than 99% purified bovine brain tubulin (phospho-cellulose purified tubulin by the method of Lee *et al.* 1978), DEAE-Cellulose purified Hela S3 Cell line tubulin (Weatherbee *et al*. 1980), and DEAE-Cellulose purified yeast and recombinant yeast tubulin (Davis *et al*. 1993).

### (2) Modified Forms of Tubulin

There are currently several types of chemically and enzymatically modified tubulins reported in the literature. Modified tubulins include biotinylated, fluorescent, tyrosinylated, non-tyrosinylated, acetylated, caged fluorescence and fluorescent analog derivatives. The lyophilization procedure described in Section (3) below has been tested on biotinylated and fluorescent derivatives. These derivatives are made stable by the lyophilization process (See Fig. 12), and, thus, have an increased shelf life as compared to non-lyophilized samples.

### (3) Lyophilization Procedure

The process of producing lyophilized active tubulin of the present invention can be applied to all isotypes, types, modified forms, recombinant forms, and all purity levels of tubulin since it has been performed on MAP-rich bovine brain tubulin, 70% pure and greater than 99% purified bovine brain tubulin, DEAE-Cellulose purified Hela S3 Cell line tubulin, DEAE-Cellulose purified yeast and recombinant yeast tubulin (70% - 95% purity). The activity of the lyophilized tubulin product is extremely high, *i.e*., greater than 95% activity.

Tubulin is purified by one of the methods described in Section (1) above by successive cycles of polymerization and depolymerization. Active tubulin will polymerize to form microtubules which are separated from non-polymerized tubulin by differential sedimentation centrifugation which sediments the microtubules into a pellet at the bottom of the centrifuge tube. The supernatant is then removed and the tube containing the pellet is placed on ice. The pellet is then resuspended in a lyophilization buffer at concentrations between 1µg/ml to 200 mg/ml and placed into a vessel for lyophilization.

Tubulin at 0.2 to 50 mg/ml is polymerized by incubating at a temperature from 4°C to 45°C for 1 to 500 minutes, and more preferably, at a temperature of 37°C. Preferably, the number of cycles of polymerization can be 1 to 6, and more preferably, about 2 to 3 for higher yields. The preferred temperature gradient for polymerization is usually less than 5 minutes from the low temperature to the high temperature. Polymerization at the increased temperature is preferably, from 1 to 500 minutes, more preferably, from 20 to 120 minutes, and most preferably, at 45 minutes. The concentration of tubulin during polymerization is preferably, between 0.2 to 50 mg/ml, more preferably, between 0.5 to 20 mg/ml, and most preferably, at 5 mg/ml.

Preferably, the temperature for centrifugation is between 15°C to 45°C, and more preferably, the temperature is 37°C. Preferably, the sample is centrifuged at 5000 x g to 500,000 x g, more preferably, 30,000 x g to 200,000 x g, and most preferably, at 100,000 x g. The period of centrifugation is preferably, for 5 to 5000 minutes, more preferably, for 10 to 1000 minutes and, most preferably, for 30 minutes. The supernatant is removed by decanting and the tube containing the pellet is placed on ice.

Prior lyophilization methods for tubulin have been unsuccessful. The present invention produces highly active lyophilized tubulin and is applicable to a wide range of uses. This invention disclosed herein illustrates that tubulin that is lyophilized at higher concentrations is more active than tubulin lyophilized at lower concentrations. It was determined that there was a 40% loss in activity for tubulin at 3 mg/ml compared to 20 mg/ml. Thus, the concentration at which the pellet is resuspended prior to lyophilization is very critical. Preferably, pellet resuspension can be at a protein concentration of 1µg/ml to 200 mg/ml, more preferably, between 0.5 to 50 mg/ml, and most preferably, at 20 mg/ml.

The tubulin lyophilization solution includes a buffer, a sugar, a carbohydrate polymer, a nucleotide, and a substitute protein. Buffers include, but are not limited to, PIPES, MES, Tris buffer and phosphate buffer. Preferably, the buffer is PIPES at a concentration of 15 mM, pH 6.9. Sugars include but are not limited to, sucrose, glucose, maltose and galactose. Preferably, the sugar is sucrose at 5% w/v. Carbohydrate polymers include, but are not limited to, dextran, polyethylene glycol and ficoll. Preferably, the carboydrate polymer is FICOLL™ (400K) at 1% w/v. Salts include but are not limited to, MgCl₂, MnCl₂, CaCl₂, and magnesium acetate. Preferably, the salt is MgCl₂ at 0.5 mM. Nucleotides include but are not limited to, adenosine triphosphate (ATP), guanosine triphosphate (GTP), and guanosine diphosphate (GDP). Substitute proteins include, but are not limited to, Bovine Serum Albumin (BSA) and Immunoglobulins like IgG. These substitute proteins can substitute for up to 50% of tubulin. Alternatively, the pellet may also be suspended in distilled water alone for a semi-stable, less than optimal formulation.

The pellet is preferably air-dried or frozen. Lyophilization is conducted preferably at a temperature between -200°C to 60°C, more preferably -45°C to 30°C, and most preferably at -40°C for frozen samples and 4°C for air-dried liquid samples. The water content (v/v) in the sample is preferably between 0% to 20%, more preferably between 0.2% to 5%, and most preferably less than 3%. Lyophilization is preferably performed at a vacuum pressure between 76 torr to 1 milli-torr, more preferably between 10 torr to 20 milli-torr and most preferably at 100 milli-torr.

### (4) Vessels for Lyophilization

Different applications for lyophilized tubulin require different vessels for lyophilization of tubulin. The vessels include, but are not limited to, single vials for all applications, wells in 96-well, 384-well, 864-well and higher well plates, wells and walls of the wells in 96-well, 384-well, 864-well and higher well plates, glass slides, solid supports, dip sticks, filters, frozen liquid drops, and any micro or nano-sized reaction chambers that may be available in the future.

### (5) Storage of Lyophilized Tubulin

After lyophilization the product can be stored at -189°C to 37°C with desicant for greater than one year (See Fig. 3) which can be extrapolated to greater than five years at 4°C. Prior storage methods involved freezing tubulin solution in liquid nitrogen and storing at -70°C (Shelanski *et al.* 1973). Storage at -70°C is unsuitable for high through-put screening and other uses because retrieving the vials requires dexterity that cannot be automated easily. Thus, the lyophilization and subsequently, the storage methods of the present invention offer significant advantages over the methods described in the prior art.

### (6) Reconstitution of Tubulin

Present reconstitution methods involve reconstitution of tubulin that is either impure, or has variable or no activity. The first aspect of the present invention may further include a novel reconstitution method that maintains high activity of the protein. Reconstitution of the pellet is conducted with a standard tubulin buffer that includes a buffer, a salt, a triphosphate nucleotide, a calcium chelator and a water exclusion agent that also promotes microtubule assembly. Buffers include, but are not limited to, PIPES, MES, Tris buffer and phosphate buffer. Preferably, the buffer is PIPES at a concentration of 80 mM. Salts include but are not limited to, MgCl₂, MnCl₂, CaCl₂, and magnesium acetate. Preferably, the salt is MgCl₂ at 1.0 mM. Triphosphate nucleotides include but are not limited to, GTP, ATP, GMP and GDP. Preferably, the triphosphate nucleotide is GTP at 1.0 mM. The calcium chelator is preferably EGTA. Water exclusion agents include, but are not limited to, sucrose, glucose, poly-ethylene glycol and glycerol. Preferably, the water exclusion agent is glycerol at a concentration of 0% to 10%.

### (7) Testing the Biological Activity of Tubulin

The third aspect of this invention is an assay by which to test the biological activity of lyophilized active tubulin.

**Microtubule Polymerization Assay**: In this assay lyophilized active tubulin obtainable by the method of the first aspect is warmed to 37°C in a spectrophotometer in the presence of a reconstitution buffer such as GTP. Over time, the tubulin polymerizes to form microtubules which refract light at 340 nm thus increasing the optical density. The optical density is proportional to the concentration of microtubule polymers, where 1.0 O.D.₃₄₀ₙₘ over 1 cm pathlength = 5.5 mg/ml microtubule polymer. Inactive tubulin will not show an increase in optical density over a 60 minute time period. This assay can be used to compare lyophilized formulations of tubulin to determine activity (e.g. Fig. 6).

A second test to determine the formation of bonafide microtubules involves placing the tubulin microtubule mixture on ice to see if the microtubules will depolymerize and reduce their measured optical density to the original value. The microtubule mixture is then reheated to 37°C to polymerize again. The lyophilized active tubulin performs this standard assay very well. Thus, these assays can be used to test the activity of different preparations of tubulin, to test the efficacy of anti-tubulin ligands, anti-tubulin antibodies, etc., and to test the stability of lyophilized active tubulin.

**Testing the stability of Lyophilized Active Microtubules**: Microtubules can be stabilized by the addition of paclitaxel. Paclitaxel is added to a solution of microtubules which is then lyophilized under the optimal conditions described in the first aspect of the present invention (see Fig. 14). These lyophilized microtubules are then used for high through-put screening for microtubule motor ligands, *e.g*., kinesin, since microtubules are the substrate for molecular motor activity.

### (8) High Through-put Screening

The lyophilization procedure of the first aspect may be conducted on tubulin in 96-well plates to show the applicability of this method to high through-put screening. Paclitaxel is added to the lyophilized active tubulin as a positive control since it promotes microtubule formation. Nocodozole is similarly used as a negative control since it depolymerizes microtubules. Substances that may potentially have an effect on microtubule polymerization can be assayed by this method in which the optical density was measured at 340 nm over 60 minutes at 15-45°C, preferably 24°C MAP-rich or 37°C pure tubulin (e.g., as an indication of microtubule polymerization). These substances include, but are not limited to, prepared plant extracts, tubulin ligands, synthetic molecules, molecules prepared from a combinatorial library, small organic molecules, molecular motor proteins, microtubule associated proteins, proteins that interact with microtubules and/or tubulin like kinesins, dyneins, MAPs, MAP2s, onc18, XKCM-1, CENP-E, and their homologues, GTPase activated or fluorescent analogs, or any other proteins that use lyophilized and reconstituted microtubules or tubulin as their substrates. The method of preparation described here creates a very accurate assay, the coefficients of variation for the nucleation, polymerization and steady state phases of microtubule polymerization are 13.6, 7.5, and 9.2 respectively.

### (9) Diagnostic Applications with Lyophilized Active Tubulin

In the fourth aspect of this invention, lyophilized active tubulin can be used for the diagnostic application of measuring anti-tubulin ligands that include, but are not limited to, taxol (paclitaxel) and mebendazole. The assay may be used to measure these ligands in body fluids, fermentor broths, and in steps of synthesis of ligands. The assay may be conducted in a vessel that includes, but is not limited to, a 96-well plate.

### (10) Therapeutic Applications with Lyophilized Active Tubulin

Lyophilized active tubulin may also be used for therapeutic applications. Lyophilized active tubulin can be used in conjunction with or without tubulin ligands in a complex for therapeutic purposes. Paclitaxel stabilized microtubules can be made by reconstitution of lyophilized active tubulin into taxol containing buffer and encapsulating them into liposomes. The liposomes will serve as the vehicle of delivery for the slow drug delivery of tubulin ligands.

This invention is illustrated in the examples which follow. The examples are set forth to give an understanding of the invention, but are not intended to, and should not be construed to limit in any way the invention as set forth in the claims which follow thereafter.

### Example 1. Determining the Optimal conditions for Lyophilization of Tubulin

The temperature and vacuum pressure at which lyophilization was conducted was optimized by studying the effects on tubulin competence after lyophilization. Tubulin was suspended in the preferred lyophilization buffer at 20 mg/ml. Lyophilization was conducted at -40°C, -10°C, 4°C, 10°C, 24°C, 37°C and 60°C. Upon completion of lyophilization, the samples were stored at 37°C for one week prior to assaying for polymerization activity. Frozen (rapid freezing in liquid nitrogen) and liquid forms of the preparation were tested over the same temperature profile.

The liquid form froze quickly after application of the vacuum at temperatures below 10°C which stabilized the protein. At 24°C, 37°C, and 45°C the liquid samples boiled vigorously and over-flowed the vessel (96-well plate).

The frozen form at -40°C, -10°C, 4°C and 10°C the samples stayed as a frozen solid (above 0°C the evaporation of water from the samples reduces their energy, which makes them cooler than the surroundings). These samples reconstituted well and polymerized well. However, there was a slight reduction in activity compared to the air-dried sample. At 24°C, 37°C and 45°C the frozen samples turned liquid and boiled the contents, over flowing the vessel.

### Example 2. Effect of Protein, buffer, sugar, carbohydrate polymer, salt and triphosphate nucleotide on Tubulin lyophilization

The optimal formulation was derived from the general tubulin buffer (80mM Pipes pH6.9, 1mM MgCl₂, 1mM EGTA and 1mM GTP (Brinkley et al. 1978). The components were adjusted as described in Fig. s 1 and 2 with the addition of a freezing protectant (sugar) and a molecular motion inhibitor (long chain carbohydrate). ,

The constituents of the preferred buffer were tested for their affects on tubulin activity after lyophilization by using the polymerization assay. The tubulin was suspended in the preferred lyophilization buffer at 20 mg/ml unless otherwise stated in Fig. 1. This tubulin solution served as the control. The samples were stored at 37°C for one week prior to assaying for polymerization activity. Only GTP had a significant short term effect on stability as shown in Fig. 1.

### Example 3. Effect of buffer, calcium chelator, salt, water exclusion agent and triphosphate nucleotide on Tubulin reconstitution

The constituents of the optimal reconstitution buffer were tested for their effects on tubulin activity after lyophilization by using the polymerization assay. 99% pure tubulin purified from bovine brain was suspended at 20 mg/ml in lyophilization buffer. The samples were stored at 37°C for one week prior to assaying for polymerization activity. Reconstitution was done in the optimal buffer as a control and buffer formulations as indicated below and in Fig. 2. It was determined that GTP, phosphate and Tris buffer all had a significant effect on activity.

The reconstitution buffers tested are as follows:
- G-PEM (Control):: 80 mM PIPES, pH 6.9, 1.0 mM MgCl₂, 1.0 mM GTP, 0.5 mM EGTA and 10% glycerol
- PEM:: 80 mM PIPES, pH 6.9, 1.0 mM MgCl₂, 0.5 mM EGTA and 10% glycerol
- G-PBS:: 50 mM Phosphate, pH 6.9, 50 mM NaCl, 1.0 mM MgCl₂, 1.0 mM GTP, 0.5 mM EGTA and 10% glycerol
- PBS:: 50 mM Phosphate, pH 6.9, 50 mM NaCl, 1.0 mM MgCl₂, 0.5 mM EGTA and 10% glycerol
- G-MEM:: 100 mM MES, pH 6.9, 1.0 mM MgCl₂, 1.0 mM GTP, 0.5 mM EGTA and 10% glycerol
- MEM:: 100 mM MES, pH 6.9, 1.0 mM MgCl₂, 0.5 mM EGTA and 10% glycerol
- G-TEM:: 80 mM Tris-HCl, pH 6.9, 1.0 mM MgCl₂, 1.0 mM GTP, 0.5 mM EGTA and 10% glycerol
- TEM:: 80 mM Tris-HCl, pH 6.9, 1.0 mM MgCl₂, 0.5 mM EGTA and 10% glycerol

### Example 4. Comparison of Frozen vs. Air-dried study for Lyophilized Active Tubulin

Polymerized tubulin was pelleted as described in Example 1 and resuspended in the preferred lyophilization formulation described in Example 2 at concentrations of 3 mg/ml and 20 mg/ml respectively. One milligram of each sample was drop frozen in liquid nitrogen in an eppendorf tube, and one milligram of each sample was placed in eppendorf tubes over ice. The samples were then lyophilized by the preferred method for frozen and air-dried liquid samples as described in Example 1. Samples were reconstituted as described in Example 3 and polymerized at 37°C for 40 minutes. The maximum O.D._{340 nm} was compared between samples.

At a resuspension concentration of 3 mg/ml before lyophilization, the frozen sample polymerized approximately 44% less than the air-dried sample. At a resuspension concentration of 20 mg/ml before lyophilization, the frozen sample polymerized approximately 7% less than the air-dried sample. Results are shown in Fig. 3

### Example 5. Testing the biological activity and stability of Tubulin

The biological activity of tubulin is tested in two ways. In the first assay a solution of tubulin is warmed to 37°C in a spectrophotometer in the presence of GTP. Over time, the tubulin polymerizes to form microtubules which refract light at 340 nm thus increasing the optical density. The optical density is proportional to the concentration of microtubule polymers, where 1.0 O.D.₃₄₀ₙₘ over 1 cm pathlength = 5.5 mg/ml microtubule polymer. Low potency tubulin (Sigma Chemical Company, St. Louis, MO, Catalog. No. T4925) will show a small increase in optical density over a 60 minute time period (See Fig. 6). This assay can be used to compare the activity of different lyophilized formulations of tubulin.

In the second assay, the formation of bonafide microtubules is studied by placing the tubulin microtubule mixture on ice to see if the microtubules will depolymerize and reduce their measured optical density to the original value. The microtubule mixture is then reheated to 37°C to polymerize again. The lyophilized active tubulin of the present invention performs this standard assay very well, whereas inactive tubulin (Sigma Chemical Company, St. Louis, MO, Catalog. No. T4925) does not depolymerize and polymerize as described. Thus, these assays can be used to test the activity of different preparations of tubulin, to test the efficacy of anti-tubulin ligands, anti-tubulin antibodies etc.

The stability of lyophilized tubulin preparations was tested by the polymerization assay. Fourteen preparations of lyophilized tubulin were prepared as described in Example 1. One plate was placed at 4°C, one at 24°C, and the other at 37°C. After 0 to 370 days, duplicate samples were tested by the same polymerization assay for activity. The lyophilized tubulin of the present invention stored at 4°C, 24°C, and 37°C was completely stable and predicted to retain polymerization activity for more than one year at room temperature (See Fig. 4).

### Example 6. Comparison of microtubule formation between lyophilized and fresh tubulin

Active tubulin was lyophilized in single vials and reconstituted with G-PEM. After incubation at 37°C for 30 minutes (See Fig. 6), it was determined that lyophilized active tubulin forms similar microtubules as seen under the electron microscope as those formed from freshly prepared tubulin (See Fig. 7). This indicates that lyophilized active tubulin is a high quality reagent suitable for drug discovery, research, therapeutics and diagnostic uses. T4925 tubulin, from Sigma Chemical Co., formed very few and irregular microtubules by the same procedure (See Fig. 7).

### Example 7. Comparison of lyophilized tubulin of the present invention with lyophilized tubulin, Catalog No. T4925, Sigma Chemical Company, St. Louis, MO.

A comparative study was done with the lyophilized tubulin of the present invention with lyophilized tubulin sold by Sigma Chemical Company, St. Louis, MO (Catalog No. T4925, Lot No. 87H4024) which is approximately 30-40% pure tubulin (See Fig. 5), hereafter referred to as the Sigma tubulin. The Sigma tubulin has approximately 30% denatured protein as determined by solubilization of the preparation in double distilled water and centrifugation at 25,000 x g at 4°C for 30 minutes as per the manufacturer's protocol. The protein pellet (denatured protein fraction) contained 30% of the total protein. As a comparison, the tubulin of the present invention had less than 5% denatured protein.

The supernatant was then warmed to 37°C to initiate polymerization. The optical density of the Sigma tubulin measured kinetically over time showed little if any polymerization. The optical density measured at 340 nm was very small, less than 0.01 O.D.₃₄₀ₙₘ (See Fig. 6). The same concentration of lyophilized tubulin of the present invention polymerized to greater than 85% (O.D. ₃₄₀ₙₘ with 0.8 cm pathlength = 0.62). This experiment was repeated thrice with similar results. Thus, the Sigma tubulin is a very low activity preparation of tubulin that was lyophilized by a non-optimal procedure. The components of the Sigma tubulin are 7.5 mg impure protein, 0.1 M MES buffer (pH 6.5), 1 mM EGTA, 0.5 mM MgCl₂, 0.1 mM EDTA, 2.5 M glycerol. Glycerol is a non-lyophilizing liquid component. It is believed that the Sigma tubulin is inactive due to the presence of glycerol which increases protein mobility and thus increases the rate of thermal denaturation, EGTA (a calcium chelator that also binds to Mg₂+ at high concentrations, as is present in the lyophilized state), and EDTA (a Mg₂+ chelator that binds two moles of Mg₂+ per mole of chelator). Since Mg₂+ is required for tubulin stability, the presence of these Mg₂+ binding substances affects stability of tubulin. The Sigma tubulin is not available in a 96-well or any other format and would not be suitable for research, drug screening, diagnostics and therapy with active tubulin.

The activity of the Sigma tubulin was compared with the tubulin of the present invention by observing microtubule formation under the electron microscope. Microtubules can be observed under the electron microscope as striated cylinders. The tubulin of the present invention forms microtubules with characteristic appearance (compare Fig. s 7a and 7b), whereas the Sigma tubulin had very few microtubules which were irregular due to aggregates of denatured protein (compare Fig. s 7a and 7c).

### Example 8. Drug Screening for tubulin ligands using lyophilized active tubulin

The lyophilization procedure is conducted on tubulin in 96-well plates to show the applicability of this method to high through-put screening. MAP-rich tubulin (100 µg/well) was lyophilized under the optimal conditions described in Example 1, in a 96-well plate. Ninety-six wells in a 96-well plate of lyophilized tubulin can be simultaneously reconstituted and their polymerization measured (See Fig. 8). The coefficient of variation for this assay is 16%, 13% and 10% for single, duplicate and triplicate assays, respectively. In a similar manner, using an 8-channel hand held pipettor, various plant extracts were prepared in G-PEM buffer at 4°C. Controls were added to the assay. Paclitaxel was added to the lyophilized active tubulin as a positive control since it promotes microtubule formation. Nocodozole was similarly used as a negative control since it depolymerizes microtubules. The optical density was measured at 340 nm over 60 minutes at 24°C as an indication of microtubule polymerization (See Fig. 9). It was observed that paclitaxel and nocodozole have enhancing and negative effects as expected, where paclitaxel promotes microtubule formation and nocodozole depolymerizes microtubules. The various nocodozole solutions had various activities (See Fig. 9) which indicates that these extracts contain chemicals that affect microtubule polymerization. Chemicals detected by similar methods may be useful for therapeutic drug development.

### Example 9. Diagnostic screening of anti-tubulin ligands

Paclitaxel and nocodozole, anti-tumor agents, were assayed in G-PEM buffer with lyophilized active tubulin. In this experiment, MAP-rich tubulin was lyophilized at 100 µg/well in a 96-well plate with the optimal procedure as described in Example 1. Solutions of paclitaxel or nocodozole at various concentrations were pipetted into individual wells at 100 µl per well. The optical density was measured at O.D.₃₄₀ₙₘ over 20 minutes at 24°C. An increased rate of polymerization was observed as indicated by an increased optical density with increasing concentrations of paclitaxel (See Fig. 10). This increase was proportional to log₁₀ [concentration of Paclitaxel]. A decrease in the rate of polymerization as indicated by a decreased optical density with increasing concentrations of nocodozole was observed (See Fig. 11). This decrease was proportional to 1/log₁₀ [concentration of nocodozole].

### Example 10. Preparation of paclitaxel stabilized microtubules

Greater than 99% purified tubulin was polymerized in a solution made up of 3 mg/ml tubulin, 80 mM PIPES, pH 6.9, 1.0 mM MgCl₂, 1.0 mM GTP, 0.5 mM EGTA and 1.0 µM paclitaxel. The solution was incubated at 37 °C for 30 minutes. Upon incubation, 10 µM paclitaxel was added and mixed in. This was incubated for a further 10 minutes and either centrifuged to pellet the microtubules or used directly in the next step. Samples were checked for intact microtubules by observation under the electron microscope. Samples were then prepared for lyophilization by adding 0.5% Ficoll and 2.5% sucrose. Samples were lyophilized in a 96-well plate under the preferred conditions described in example 2. The samples were then stored at 37°C for one week. Samples were reconstituted in G-PEM and small volumes taken for electron microscopy observation. It was determined that microtubules were present before and after lyophilization indicating that microtubules can be successfully lyophilized with this procedure (See Fig. 16).

## Claims

1. A method for the isolation of lyophilized active tubulin comprising the steps of:
(a) running multiple cycles of polymerisation and depolymerisation of tubulin;
(b) conducting differential sedimentation centrifugation to create a pellet of active tubulin;
(c) resuspending the pellet in a lyophilization buffer at a tubulin concentration of between 1 µg/ml and 200 mg/ml, the lyophilization buffer comprising (a) a buffer, a sugar, a carbohydrate polymer, a salt, a nucleotide and a protein or (b) distilled water;
(d) freezing the resuspended pellet in liquid nitrogen;
(e) lyophilizing the frozen resuspended pellet at a temperature of 10 °C or below.

2. The method of claim 1, wherein the pellet is resuspended in the lyophilization buffer at a tubulin concentration of 20 mg/ml.

3. The method of claim 1 or claim 2, further comprising reconstituting the lyophilized tubulin in a reconstitution buffer.

4. The method of claim 3, wherein the reconstitution buffer comprises of:
(a) buffer;
(b) a salt;
(c) a triphosphate nucleotide;
(d) a calcium chelator; and
(e) a water exclusion agent.

5. The method of any preceding claim, wherein said lyophilization is conducted in multi-well plates for specific use in through-put screens.

6. The method of any preceding claim, wherein said lyophilization is conducted in lyophilization vessels selected from the group consisting of single vials for all applications, wells in 96-well, 384-well, 864-well and higher well plates, wells and walls of the wells in 96-well, 384-well, 864-well and higher well plates, glass slides, solid supports, dip sticks, filters, frozen liquid drops, any micro or nano-sized reaction chambers, and combinations thereof

7. The method of any preceding claim, wherein the tubulin is in the form of microtubules.

8. A lyophilized active tubulin protein obtainable by the method of claim 1 or claim 2, which polymerises upon reconstitution.

9. A lyophilized active tubulin protein as claimed in claim 8, wherein the tubulin is a modified tubulin, a recombinant tubulin, a cell line derived tubulin, or an isolated tubulin from tissues and organs.

10. A microtubule polymerization assay for lyophilized active tubulin comprising the following steps:
(a) warming lyophilized active tubulin protein obtainable by the method of claim 1 or claim 2 to 37 °C in a spectrophotometer in the presence of a reconstitution buffer; and
(b) measuring the optical density at O.D. _{340 nm} over a time period.

11. A diagnostic assay for tubulin ligands comprising the following steps:
(a) adding the tubulin ligand in reconstitution buffer to a lyophilized tubulin protein obtainable by the method of claim 1 or claim 2; and
(b) measuring the optical density at O.D. _{340 nm} over a time period.

12. A drug discovery process comprising the following steps:
(a) reconstituting lyophilized active tubulin obtainable by the method of claim 1 or claim 2 in a reconstitution buffer;
(b) incubating the reaction (at 24°C or 37 °C) and measuring at O.D. 340nm over 60 minutes; and
(c) comparing control and test samples for differences in polymerization.

13. A drug discovery process comprising the following steps:
(a) reconstituting lyophilized active tubulin obtainable by the method of claim 1 or claim 2 in a vessel that supports high through-put drug screening;
(b) incubating the reaction and measuring at O.D. _{340 nm}; and
(c) comparing control and test samples for differences in polymerization kinetics.

14. A method for detecting the presence of an analyte in solution comprising the following steps:
(a) adding the analyte to lyophilized tubulin protein obtainable by the method of claim 1 or claim 2; and
(b) measuring the optical density at O.D. _{340 nm} over a time period.

15. A method of drug discovery which uses lyophilized active tubulin obtainable by the method of claim 1 or claim 2 as a component of a lyophilized mixture of proteins.

## Patentansprüche

1. Verfahren zum Isolieren von lyophilisiertem aktivem Tubulin, welches die Schritte aufweist:
(a) Ausführen von mehreren Zyklen von Polymerisation und Depolymerisation von Tubulin;
(b) Ausführen einer differentiellen Sedimentaionszentrifugation, um ein Pellet von aktivem Tubulin zu erzeugen;
(c) Resuspendieren des Pellets in einem Lyophilisierungspuffer zu einer Tubulinkonzentration von zwischen 1µg/ml und 200 mg/ml, wobei der Lyophilisierungspuffer (a) einen Puffer, einen Zucker, ein Kohlenhydratpolymer, ein Salz, ein Nukleotid und ein Protein oder (b) destilliertes Wasser aufweist;
(d) Gefrieren des resuspendierten Pellets in flüssigem Stickstoff;
(e) Lyophilisieren des gefrorenen resuspendierten Pellets bei einer Temperatur von 10 °C oder niedriger.

2. Verfahren nach Anspruch 1, wobei das Pellet im Lyophilisierungspuffer zu einer Tubulinkonzentration von 20 mg/ml resuspendiert wird.

3. Verfahren nach Anspruch 1 oder Anspruch 2, welches weiters das Rekonstituieren des lyophilisierten Tubulins in einem Rekonstitutionspuffer.

4. Verfahren nach Anspruch 3, wobei der Rekonstitutionspuffer enthält
(a) Buffer;
(b) Ein Salz;
(c) Ein Triphosphatnukleotid;
(d) Einen Kalziumchelatbildner; und
(e) Ein Wasser-Ausschussmittel.

5. Verfahren nach einem der vorangehenden Ansprüchen, wobei die Lyophilisierung in Mehrkammer-Platten für die spezifische Verwendung in Durchlauf-Sceenings erfolgt.

6. Verfahren nach einem der vorangehenden Ansprüchen, wobei die Lyophilisierung in Lyophilisierungsgefäßen durchgeführt wird, die ausgewählt sind, aus der Gruppe aus einzelnen Phiolen für alle Anwendungen, Kammern in 96-Kammer, 384-Kammer, 864-Kammer oder höhere Kammer-Platten, Kammern und Wände der Kammern in 96-Kammer, 384-Kammer, 864-Kammer oder höhere Kammer-Platten, Glas-Objektträger, Feststoffträger, Tauchstäbe, Filter, gefrorene flüssige Tropfen, jede mikro- oder nanogroße Reaktionskammer, und Kombinationen davon.

7. Verfahren nach einem der vorangehenden Ansprüche, wobei das Tubulin in Form von Mikrotubuli ist.

8. Ein lyophilisiertes aktives Tubulin, das durch das Verfahren nach Anspruch 1 oder Anspruch 2 erhältlich ist, welches bei der Rekonstitution polymerisiert.

9. Ein lyophilisiertes aktives Tubulinprotein nach Anspruch 8, wobei das Tubulin ein modifiziertes Tubulin, ein rekombinantes Tubulin, ein aus einer Zelllinie stammendes Tubulin, oder ein aus Geweben oder Organen isoliertes Tubulin ist.

10. Mikrotubulus-Polymerisationsuntersuchung für lyophilisiertes aktives Tubulin, welche die folgenden Schritte aufweist:
(a) Wärmen des lyophiliserten aktiven Tubulinproteins, welches durch das Verfahren nach Anspruch 1 oder Anspruch 2 erhältlich ist, in einem Spektrophotometer in der Gegenwart eines Rekonstitutionspuffers auf 37 °C; und
(b) Messen der optischen Dichte bei O.D. 340 über ein Zeitperiode.

11. Diagnostische Untersuchung für Tubulinliganden, welche folgende Schritte aufweist:
(a) Hinzufügen des in Rekonstitutionspuffer befindlichen Tubulinliganden zu lyophilisiertem Tubulinprotein, welches durch das Verfahren nach Anspruch 1 oder 2 erhältlich ist; und
(b) Messen der optischen Dichte bei O.D. 340 nm über eine Zeitperiode.

12. Arzneimittel-Auffindungssverfahren, welches folgende Schritte aufweist:
(a) Rekonstituieren des lyophilisierten aktiven Tubulins, welches durch das Verfahren nach Anspruch 1 oder Anspruch 2 erhältlich ist in einem Rekonstitutionspuffer;
(b) Inkubieren der Reaktion (bei 24 °C oder 37 °C) und Messen O.D. 340 nm für 60 Minuten; und
(c) Vergleichen der Kontroll- und Testprobe auf Unterschiede in der Polymerisation.

13. Arzneimittel- Auffindungssverfahren, welches folgende Schritte aufweist:
(a) Rekonstituieren des lyophilisierten aktiven Tubulins, welches durch das Verfahren nach Anspruch 1 oder Anspruch 2 erhältlich ist, in einem Gefäß, welches das Hochdurchlauf-Sceenen unterstützt;
(b) Inkubieren der Reaktion und Messen der O.D. 340 nm; und
(c) Vergleichen der Kontroll- und Testprobe auf Unterschiede in der Polymerisation.

14. Verfahren zum Ermitteln des Vorhandenseins eines Analyten in Lösung, welches die folgenden Schritte aufweist:
(a) Hinzufügen des Analyten zu lyophilisertem Tubulinprotein, welches durch das Verfahren nach Anspruch 1 oder Anspruch 2 erhältlich ist; und
(b) Messen der optischen Dichte bei O.D 340nm über eine Zeitperiode.

15. Verfahren zur Arzneimittel-Auffindung, welches lyophilisiertes aktives Tubulin, welches durch das Verfahren nach Anspruch 1 oder Anspruch 2 erhältlich ist, als ein Bestandteil einer lyophilisierten Proteinmischung verwendet.

## Revendications

1. Procédé pour l'isolement de tubuline active lyophilisée, comprenant les étapes suivantes :
(a) réalisation de multiples cycles de polymérisation et dépolymérisation de la tubuline;
(b) réalisation d'une centrifugation à sédimentation différentielle pour créer un culot de tubuline active;
(c) remise en suspension du culot dans un tampon de lyophilisation, à une concentration de tubuline de 1 µg/ml à 200 mg/ml, le tampon de lyophilisation comprenant (a) un tampon, un sucre, un polymère d'hydrate de carbone, un sel, un nucléotide et une protéine ou (b) de l'eau distillée;
(d) congélation dans de l'azote liquide du culot remis en suspension;
(e) lyophilisation du culot remis en suspension congelé, à une température de 10°C ou moins.

2. Procédé selon la revendication 1, dans lequel le culot est remis en suspension dans le tampon de lyophilisation à une concentration de tubuline de 20 mg/ml.

3. Procédé selon la revendication 1 ou la revendication 2, comprenant en outre la reconstitution de la tubuline lyophilisée dans un tampon de reconstitution.

4. Procédé selon la revendication 3, dans lequel le tampon de reconstitution est composé :
(a) d'un tampon;
(b) d'un sel;
(c) d'un nucléotide triphosphate;
(d) d'un chélateur de calcium; et
(e) d'un agent hydrofuge.

5. Procédé selon l'une quelconque des revendications précédentes, dans lequel ladite lyophilisation est réalisée dans des plateaux multi-puits pour utilisation spécifique dans des cribles de débit.

6. Procédé selon l'une quelconque des revendications précédentes, dans lequel ladite lyophilisation est réalisée dans des récipients de lyophilisation sélectionnés dans le groupe constitué de flacons uniques pour toutes les applications, puits dans des plateaux à 96 puits, 384 puits, 864 puits et plus, puits et parois des puits dans des plateaux à 96 puits, 384 puits, 864 puits et plus, lames de verre, supports solides, bandelettes réactives, filtres, gouttes de liquide congelé, chambres de micro- ou nano-réaction quelconques, et leurs combinaisons.

7. Procédé selon l'une quelconque des revendications précédentes, dans lequel la tubuline est sous la forme de microtubules.

8. Tubuline active lyophilisée pouvant être obtenue par le procédé selon la revendication 1 ou la revendication 2, et qui se polymérise à la reconstitution.

9. Tubuline active lyophilisée selon la revendication 8, dans laquelle la tubuline est une tubuline modifiée, une tubuline recombinée, une tubuline dérivée d'une lignée cellulaire ou une tubuline isolée de tissus et d'organes.

10. Essai de polymérisation en microtubules de tubuline active lyophilisée, comprenant les étapes suivantes :
(a) chauffage à 37°C de protéine tubuline active lyophilisée pouvant être obtenue par le procédé selon la revendication 1 ou la revendication 2 dans un spectrophotomètre, en présence d'un tampon de reconstitution; et
(b) mesure de la densité optique à 340 nm pendant un certain temps.

11. Essai de diagnostic de ligands de tubuline comprenant les étapes suivantes :
(a) addition du ligand de tubuline dans un tampon de reconstitution à une protéine tubuline lyophilisée pouvant être obtenue par le procédé selon la revendication 1 ou de la revendication 2 ; et
(b) mesure de la densité optique à 340 nm pendant un certain temps.

12. Procédé de découverte de médicaments, comprenant les étapes suivantes :
(a) reconstitution de la tubuline active lyophilisée, pouvant être obtenue par le procédé selon la revendication 1 ou la revendication 2, dans un tampon de reconstitution;
(b) incubation de la réaction (à 24°C ou 37°C) et mesure de la densité optique à 340 nm pendant 60 minutes; et
(c) comparaison des échantillons d'essai et témoins pour les différences de polymérisation.

13. Procédé de découverte de médicaments, comprenant les étapes suivantes :
(a) reconstitution de la tubuline active lyophilisée, pouvant être obtenue par le procédé selon la revendication 1 ou la revendication 2, dans un récipient permettant le criblage médicamenteux à haut débit;
(b) incubation de la réaction et mesure de la densité optique à 340 nm; et
(c) comparaison des échantillons d'essai et témoins pour les différences de cinétique de polymérisation.

14. Procédé pour détecter la présence d'une substance à analyser dans une solution, comprenant les étapes suivantes :
(a) addition de la substance à analyser à la protéine tubuline lyophilisée pouvant être obtenue par le procédé selon la revendication 1 ou la revendication 2; et
(b) mesure de la densité optique à 340 nm pendant un certain temps.

15. Procédé de découverte de médicaments qui utilise la tubuline active lyophilisée pouvant être obtenue par le procédé de la revendication 1 ou de la revendication 2 comme composant d'un mélange de protéines lyophilisé.
